# EUROPEAN PATENT APPLICATION

(11) **EP 3 440 990 A1**
(43) Date of publication of application: **13.02.2019**
(21) Application number: 17185475.5
(22) Date of filing: 09.08.2017
(51) Int. Cl.: A61B 3/15, A61B 3/12, A61B 3/14

(54) **SYSTEM FOR IMAGING A FUNDUS OF AN EYE**

(71) Applicant: Koninklijke Philips N.V., 5656 AE Eindhoven (NL)
(72) Inventor: JUTTE, Petrus Theodorus, 5656 AE Eindhoven (NL); DE BRUIJN, Frederik Jan, 5656 AE Eindhoven (NL); SCHMEITZ, Harold Agnes Wilhelmus, 5656 AE Eindhoven (NL); HADDEMAN, Theodoor Bastiaan Johannes, 5656 AE Eindhoven (NL)
(74) Representative: de Haan, Poul Erik

(57) **Abstract**

There is provided a system (100) for imaging a fundus of an eye (102). The system comprises an imaging sensor (104) configured to be aimed towards the subject to acquire an image of the fundus and a light interceptor (106) comprising an aperture. The light interceptor is positioned on an optical axis between the imaging sensor and eye at a point at which an image of the pupil aligns with the aperture and is movable relative to the optical axis to selectively block light passing from the eye to the imaging sensor. The system comprises one or more processors (108) operably coupled with the imaging sensor and the light interceptor, configured to detect movement of the pupil in the image and control the light interceptor to move relative to the optical axis in response to the detected movement to maintain alignment of the image of the pupil with the aperture.

## Description

### TECHNICAL FIELD OF THE INVENTION

The invention relates to the field of optics and, in particular, to a system for imaging a fundus of an eye of a subject and a method of operating the system.

### BACKGROUND TO THE INVENTION

Various aspects of the human eye are known to reflect physical or physiological changes to, or conditions of, other parts of the body. For example, the fundus of the eye includes a light sensitive (or photosensitive), image-capturing tissue referred to as the "retina", which is known to exhibit a variety of characteristics that make it a valuable source of diagnostic information for eye-related diseases (such as glaucoma), as well as other diseases of the body (such as diabetes). The retina of the eye is known to respond to physical or physiological changes of the entire body. Some of these changes are visible or measurable, and are thus helpful in the early detection of symptoms and diagnosis diseases. The fundus of the eye also includes other parts that can provide useful information on the health of a subject, such as the optic disc, the macula, the fovea, blood vessels, nerves, and so on.

A unique feature of the fundus is that it offers a relatively obstruction-free view of various arteries and veins, without being inhibited by occluding and/or scattering layers such as the skin. This enables ophthalmologists to check for the presence of vascular anomalies, such as aneurisms and even neovascularization. Additionally, the unobstructed view on the fundus blood vessels poses an advantage in assessment of the levels of various metabolic compounds carried by the vascular system by exploiting the differences in characteristic absorption of visible and invisible ultraviolet or infrared light.

Traditionally, an ophthalmologist examines the eye using an ophthalmoscope, which provides a direct view of a fragment of the fundus under coaxial illumination from a built-in light source. Ophthalmoscopes can take various forms and can be used for direct observation of the fundus or for indirect observation of the fundus by using a relay lens that is held separately near the eye. Ophthalmoscopes for direct observation of the fundus (such as classical ophthalmoscopes or the newer "panoptic" ophthalmoscopes) are typically held relatively close to the eye and, in some instances, a portion of the ophthalmoscope may even rest softly on the orbital region of the eye for support. In contrast, ophthalmoscopes for indirect observation of the fundus can be used at a remote distance from the eye but these ophthalmoscopes still require a handheld relay lens to be held close to the eye.

Therefore, existing techniques for both direct and indirect fundus imaging (for example, using an ophthalmoscope) are obtrusive. The existing techniques are also typically only performed by trained professionals in a medical setting, which means that eyes are seldom examined for fundus symptoms other than during the occasional eye test for glasses or the Amsler test for macular degeneration or other vision problems. Consequently, markers of various diseases that may be observable in the eyes often remain undetected.

With the advances in chemical and digital photography, the ophthalmoscope has evolved into the fundus camera. The fundus camera is useful for fundus imaging in cases where only the backside of the fundus needs to be captured and not the peripheral fundus area. Some fundus cameras use a line-wise scanning method based on laser illumination. Other fundus cameras that are used to compose a fundus image include planar silicon image sensors, which are based on charge-coupled devices (CCDs), or complementary metal-oxide semiconductor (CMOS) imaging sensors.

However, as with the more traditional ophthalmoscopes mentioned earlier, the position of existing fundus cameras required for fundus imaging means that the fundus camera can be obtrusive. Moreover, the fundus camera needs to be used by trained users, namely ophthalmoscope specialists. It would be beneficial for fundus cameras to be used at larger distances from the eye such that the imaging performed using the fundus cameras is unobtrusive and can be used by untrained users. However, a main issue associated with using fundus cameras at larger working distances is that the image contrast is more limited due to unwanted reflections of light from the rim of the eye. The subject is also required to avoid moving their eye and this restriction on the freedom of movement of the subject makes existing fundus imaging systems inconvenient.

There is thus a need for an improved system for imaging a fundus of an eye of a subject.

### SUMMARY OF THE INVENTION

As noted above, a limitation with existing techniques for imaging a fundus of an eye of a subject is that, although imaging at a larger working distance is beneficial since it avoids physical contact to the subject, the image contrast is more limited at larger working distances due to unwanted reflections of light from the rim of the eye. It would thus be beneficial to provide a system in which unwanted reflections of light from the rim of the eye can be avoided.

It would thus be valuable to have an improved system for imaging a fundus of an eye of a subject, which overcomes the problems described above.

Therefore, according to a first aspect of the invention, there is provided a system for imaging a fundus of an eye of a subject. The system comprises an imaging sensor configured to be aimed towards the subject to acquire an image of the fundus of the eye of the subject and a light interceptor comprising an aperture. The light interceptor is positioned on an optical axis between the imaging sensor and the eye at a point at which an image of the pupil of the eye aligns with the aperture of the light interceptor. The light interceptor is movable relative to the optical axis to selectively block light passing through the system from the eye to the imaging sensor. The system also comprises one or more processors operably coupled with the imaging sensor and the light interceptor. The one or more processors are configured to detect movement of the pupil of the eye in the image acquired by the imaging sensor and control the light interceptor to move relative to the optical axis in response to the detected movement of the pupil of the eye to maintain the alignment of the image of the pupil of the eye with the aperture of the light interceptor.

In some embodiments, the one or more processors may be configured to control the light interceptor to maintain one or more of a lateral position of the image of the pupil of the eye in alignment with a lateral position of the aperture of the light interceptor and a longitudinal position of the image of the pupil of the eye in alignment with a longitudinal position of the aperture of the light interceptor. In some embodiments, the one or more processors may be configured to control the light interceptor to move laterally to the optical axis and/or longitudinally along the optical axis in response to the detected movement of the pupil of the eye to maintain the alignment of the image of the pupil of the eye with the aperture of the light interceptor.

In some embodiments, light passing through the system from the outside of the pupil of the eye may be blocked by the light interceptor. In some embodiments, the imaging sensor may be configured to acquire the image of the fundus of the eye of the subject by imaging the pupil of the eye through the aperture of the light interceptor.

In some embodiments, the system may further comprise a light source configured to provide an illumination beam towards the fundus of the eye of the subject. In these embodiments, a diameter of the illumination beam may be greater than a diameter of the pupil of the eye of the subject.

In some embodiments, the light interceptor may be positioned on the optical axis between the imaging sensor and the eye at the point at which the pupil of the eye is in focus in the system. In some embodiments, only light reflected from the fundus of the eye and passing through the pupil of the eye may be received at the imaging sensor. In some embodiments, the imaging sensor may be configured to acquire the image of the fundus of the eye at a remote distance from the eye.

In some embodiments, the system may further comprise one or more relay lenses positioned along the optical axis between the imaging sensor and the eye and/or one or more imaging lenses positioned along the optical axis between the imaging sensor and the eye. In some embodiments, the light interceptor may be positioned between at least one of the relay lenses and at least one of the imaging lenses.

In some embodiments, the imaging sensor may be configured to acquire a sequence of images of the fundus of the eye and combine the sequence of images of the fundus of the eye to form a single image of the fundus of the eye with a larger field of view than the individual images in the sequence.

In some embodiments, the light interceptor may be removable from the system and shaped according to a shape of the pupil of the eye of the user.

According to a second aspect of the invention, there is provided a method for operating a system to image a fundus of an eye of a subject. The method comprises acquiring, by an imaging sensor aimed towards the subject, an image of the fundus of the eye of the subject. The method also comprises detecting, by one or more processors, movement of the pupil of the eye in the image acquired by the imaging sensor and controlling, by one or more processors, a light interceptor comprising an aperture. The light interceptor is positioned on an optical axis between the imaging sensor and the eye at a point at which an image of the pupil of the eye aligns with the aperture of the light interceptor and the light interceptor is movable relative to the optical axis to selectively block light passing through the system from the eye to the imaging sensor. The light interceptor is controlled by the one or more processors to move relative to the optical axis in response to the detected movement of the pupil of the eye to maintain the alignment of the image of the pupil of the eye with the aperture of the light interceptor.

According to a third aspect of the invention, there is provided a computer program product comprising a computer readable medium, the computer readable medium having computer readable code embodied therein, the computer readable code being configured such that, on execution by a suitable computer or processor, the computer or processor is caused to perform the method described above.

According to the aspects and embodiments described above, the limitations of existing techniques are addressed. In particular, according to the above-described aspects and embodiments, an unobtrusive imaging system is provided by which imaging of a fundus of an eye of a subject can be performed at a remote distance from the eye, such that the system may operate without physical contact to the subject, whilst unwanted reflections of light (for example, from the rim of the eye) are avoided. In particular, by positioning a light interceptor on an optical axis between the imaging sensor and the eye at a point at which an image of the pupil of the eye aligns with the aperture of the light interceptor and controlling the light interceptor to move relative to the optical axis in response to a detected movement of the pupil of the eye to maintain the alignment of the image of the pupil of the eye with the aperture of the light interceptor, only the desired light contribution from the fundus is allowed to be preserved and the stray light (for example, from rim of the eye) is blocked. In this way, the image contrast that is possible at a remote distance from the eye is improved such that sharper (and thus higher quality) images can be acquired at the remote distance. The system can thus be used by untrained users, such as general practitioners, nurse practitioners, or even the subject themselves (for example, at home), thereby increasing the availability of fundus imaging. Moreover, the subject is free to move their eye during fundus imaging using the system and this freedom of movement provides more convenience for the subject.

There is thus provided an improved system for imaging a fundus of an eye of a subject, which overcomes the existing problems.

### BRIEF DESCRIPTION OF THE DRAWINGS

For a better understanding of the invention, and to show more clearly how it may be carried into effect, reference will now be made, by way of example only, to the accompanying drawings, in which:
Figure 1 is an illustration of a system for imaging a fundus of an eye of a subject according to an embodiment;
Figure 2 is an illustration of a system for imaging a fundus of an eye of a subject according to another embodiment;
Figure 3 is an illustration of a system for imaging a fundus of an eye of a subject according to an embodiment;
Figure 4 is a flow chart illustrating a method of operating a system to image a fundus of an eye of a subject according to an embodiment; and
Figures 5A and 5B are images illustrating the contrast improvements achieved by a system according to an embodiment.

### DETAILED DESCRIPTION OF THE EMBODIMENTS

As mentioned earlier, in existing systems, it is not possible to acquire (or capture) sharp images of the fundus of the eye unobtrusively, at a remote distance from the eye, without the image contrast becoming more limited due to unwanted reflections of light from the rim of the eye. The system for imaging a fundus of an eye of a subject described herein avoids unwanted reflections of light (for example, from the rim of the eye) to improve image contrast and enable sharper (and thus higher quality) images of the fundus to be acquired at a remote distance from the eye. The system described herein may also be referred to as an optical system, a fundus imaging system, or an optical fundus imaging system.

Briefly, the improved system for imaging a fundus of an eye of a subject comprises an imaging sensor configured to be aimed towards the subject to acquire an image of the fundus of the eye of the subject. The system also comprises a light interceptor. The light interceptor comprises an aperture (or a hole or an opening) and is positioned on an optical axis between the imaging sensor and the eye at a point at which an image of the pupil of the eye aligns (or corresponds, or coincides) with the aperture of the light interceptor. The light interceptor is movable relative to the optical axis to selectively block light passing through the system from the eye to the imaging sensor.

The system also comprises one or more processors (or one or more control units) operably coupled with the imaging sensor and the light interceptor. The one or more processors are configured to detect movement of the pupil of the eye in the image acquired by the imaging sensor and control the light interceptor to move relative to the optical axis in response to the detected movement of the pupil of the eye to maintain the alignment (or correspondence, or coincidence) of the image of the pupil of the eye with the aperture of the light interceptor.

By positioning (or locating) the light interceptor on the optical axis between the imaging sensor and the eye at a point at which an image of the pupil of the eye aligns with the aperture of the light interceptor and controlling the light interceptor to move relative to the optical axis in response to a detected movement of the pupil of the eye to maintain the alignment of the image of the pupil of the eye with the aperture of the light interceptor, only the desired light contribution from the fundus is allowed to be preserved and the stray light (for example, from rim of the eye) is blocked. In this way, the image contrast that is possible at a remote distance from the eye is improved such that sharper (and thus higher quality) images can be acquired at the remote distance.

In any of the embodiments described herein, the light interceptor may be moved electronically. For example, in some embodiments, the light interceptor may be moved using optical device technology, such as liquid crystal device LCD technology, digital mirror device DMD technology, or any other technology suitable for electronically moving the light interceptor. In embodiments where the light interceptor is moved using LCD or DMD technology, the light interceptor may, for example, comprise a matrix of individually controllable LCD or DMD pixel elements, which can function as an active spatial light modulator that is arranged to function as an aperture as described earlier. Alternatively or in addition, the light interceptor may be moved mechanically, for example, to extend the lateral placement of the aperture beyond the physical area of the active spatial light modulator. For example, in some embodiments, the light interceptor may be moved using a piezo coil motor, a voice coil motor, or any other component suitable for mechanically moving the light interceptor. In some embodiments where the light interceptor is moved mechanically, the mechanical movement may extend the lateral placement of the aperture of the light interceptor beyond the physical area of the aperture (for example, the active spatial light modulator mentioned earlier).

In any of the embodiments described herein, the movement of the pupil of the eye can be detected by determining the position of the eye and thus the position of the pupil of the eye. The position of the pupil of the eye can be determined by measuring the position of the pupil of the eye with respect to the optical system, such as with respect to an optical axis of the system that is between the eye of the subject and the imaging sensor of the system. In some embodiments, the eye (and, optionally, the face) of the subject can be recognized through image processing and this recognition can be applied to determine the position of the pupil of the eye. A common eye gaze tracking method is an example of a technique that can be used to determine the position of the pupil of the eye and thus to detect the movement of the pupil of the eye. The person skilled in the art will be aware of various techniques that can be used for detecting the position and movement of the pupil of the eye, many of which are based on near-infrared imaging (e.g. using invisible light in the wavelength range between 750 nm and 950 nm).

In some embodiments, the pupil may be imaged using a secondary image sensor that shares the same optical axis as the system described herein for imaging the fundus. This can be achieved, for example, by the use of a semi-transparent mirror or a dichroic mirror that splits-off a secondary light path along a secondary optical axis where the secondary image sensor is positioned such that its optical plane coincides with the plane of the light interceptor. The secondary image sensor may capture secondary images containing the anterior part of the eye, which can include the pupil. In various embodiments, the secondary images are, for example, used to estimate the center of the pupil, the coordinates of which can be used to position the aperture of the light interceptor to the corresponding position in the image plane along the optical axis of the system described herein for imaging the fundus.

Alternatively, or additionally, in various embodiments, the secondary images are, for example, used to estimate the diameter of the pupil, the value of which can be used to adjust the size of the aperture of the light interceptor to the corresponding diameter of the pupil. Alternatively, or additionally, in various embodiments, the secondary images are, for example, used to estimate the shape of the pupil, which can be approximately elliptical due to non-orthogonal observation or more complex due partial occlusion. The shape of the aperture of the light interceptor can then be adjusted to match the visible shape of the pupil. Alternatively, or additionally, in various embodiments, the proper focus of the secondary image of the pupil may be assessed to correct a longitudinal position of the light interceptor such that it always matches the focal plane of the image of the pupil along the optical axis of the system described herein for imaging the fundus.

Although the imaging sensor is described herein as being configured to acquire an image of the fundus of the eye of the subject, it will be understood that the imaging sensor can acquire one or more images (for example, any number of images) of the fundus of the eye of the subject in the manner described herein. According to any of the embodiments herein, the imaging sensor may, for example, comprise a camera, a charge-coupled device (CCD) imaging sensor, a complementary metal-oxide semiconductor (CMOS) imaging sensor, or any other imaging sensor, or any combination of imaging sensors, suitable for imaging the fundus of the eye. In some embodiments, the imaging sensor may be an RGB color imaging sensor. In other embodiments, the imaging sensor can be a monochrome imaging sensor.

The fundus of the eye referred to herein comprises the retina, the optic disc, the macula, the fovea, blood vessels, nerves, and any other features of the posterior of the eye of which a person skilled in the art will be aware. Thus, any one or more of these features of the fundus of the eye of the subject can be imaged using the system described herein.

The image of the fundus of the eye can be acquired at a remote distance from the eye using the system described herein. A remote distance from the eye referred to herein can be, for example, a distance that is larger than the distance possible to acquire suitably sharp images of the eye using existing equipment. For example, in some embodiments, the distance between the eye and the imaging sensor may be a distance equal to 10 centimeters, or a distance greater than 10 centimeters (for example, 15 centimeters, 30 centimeters, or any other distance). However, it will be understood that other distances between the eye and the imaging sensor are also possible.

In any of the embodiments described herein, one or more components of the system may be controllable by one or more of the processors of the system, which may be operably coupled to the one or more components. The one or more processors may comprise one or more processing units or one or more multi-core processors or modules that are configured or programmed to control one or more components of the system. For example, in some embodiments, the one or more processors may be configured to execute instructions stored in memory to control one or more components of the system. In other embodiments, the one or more processors may comprise logic such as a field-programmable gate array ("FPGA"), an application-specific integrated circuit ("ASIC"), or any other types of logic, or any combination of types of logic, and the logic may be configured or programmed to control one or more components of the system. Thus, the one or more processors can comprise a plurality of software and/or hardware modules that are configured to control one or more components of the system. In various embodiments, the one or more processors may be communicatively coupled with one or more remote computing devices, such as via one or more networks. The one or more networks may include one or more local area networks, wide area networks (such as the Internet), one or more personal area networks (such as Bluetooth), or similar.

In some embodiments, the system can also comprise a light source for illuminating the eye of the subject with light. For example, the light source can be configured to provide an illumination beam (or emit light) towards the eye of the subject or, more specifically, towards the fundus of the eye of the subject. In some embodiments, a diameter of the illumination beam may be greater than a diameter of the pupil of the eye of the subject. The wavelength of the light source described herein can, for example, be visible light (such as a light source configured to emit light of a wavelength in the range of 400-700 nm), visible light without light of the blue wavelength range (such as a light source configured to emit light of a wavelength in the range of 500-700 nm) or infrared IR light (such as a light source configured to emit light of a wavelength greater than 780 nm). In some embodiments, visible light without light of the blue wavelength range may be used to improve eye safety due to the possible photo-chemical eye damage that intense blue light may cause. In some embodiments, IR light may be used to improve the convenience for the subject under test and to provide a larger field of view since no pupil contraction will occur.

The system according to any of the embodiments described herein is unobtrusive. In some embodiments, the system may also be at least partially (or fully) automated.

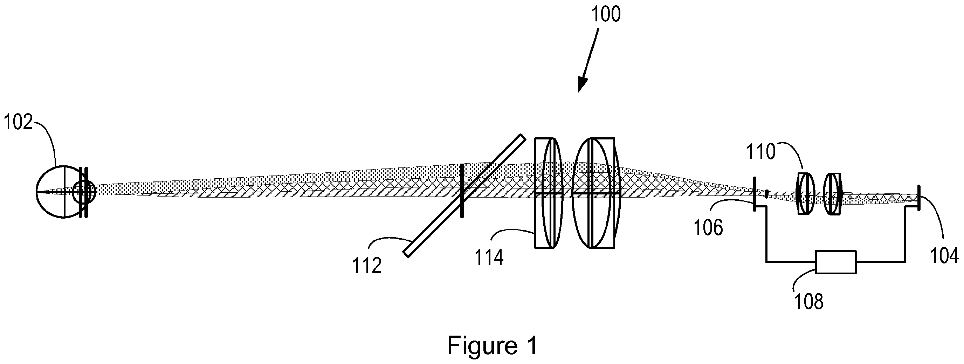

Figure 1 illustrates an example of the system 100 for imaging a fundus of an eye 102 of a subject (not depicted) according to an embodiment. Although the system will be described with reference to the arrangement of components shown in Figure 1, it will be understood that other components and arrangements of components are possible.

With reference to Figure 1, the system 100 comprises an imaging sensor 104. The imaging sensor 104 is configured to be aimed towards the subject to acquire an image of the fundus of the eye 102 of the subject. As mentioned earlier, in some embodiments, the imaging sensor 104 may be configured to acquire the image of the fundus of the eye 102 at a remote distance from the eye 102.

The system 100 also comprises a light interceptor 106. The light interceptor 106 is an opaque structure that can block light or, in other words, that can prevent the passage of light. The light interceptor 106 of the system 100 comprises an aperture (or a hole or an opening). Examples of a light interceptor 106 include, but are not limited to, a plate comprising an aperture, a diaphragm comprising an aperture, a stop comprising an aperture, an optical device (such as a liquid crystal device LCD, a digital mirror device DMD, or any other optical device) comprising an aperture, or any other component, or any combination of components, which comprise an aperture and which is suitable for intercepting light in the manner described herein.

As illustrated in Figure 1, the light interceptor 106 is positioned on an optical axis between the imaging sensor 104 and the eye 102. The light interceptor 106 is positioned on the optical axis at a point at which an image of the pupil of the eye 102 aligns (or corresponds, or coincides) with the aperture of the light interceptor 106. In some embodiments, the light interceptor 106 may be positioned on the optical axis between the imaging sensor 104 and the eye 102 at the point at which the pupil of the eye is in focus in the system 100. The point at which the pupil is in focus in the system 100 is, for example, where a light beam reflected from the eye has the smallest diameter. In some embodiments, the point at which the pupil is in focus in the system 100 is at an image of the rim of the eye 102 (or at the image plane of the rim of the eye 102).

The light interceptor 106 is movable relative to the optical axis to selectively block light passing through the system 100 from the eye 102 to the imaging sensor 104. More specifically, the light interceptor 106 prevents the passage of light, except for the light passing through its aperture. In some embodiments, light passing through the system 100 from the outside of the pupil of the eye 102 is blocked by the light interceptor 106. The imaging sensor 104 of the system 100 can be configured to acquire the image of the fundus of the eye 102 of the subject by imaging the pupil of the eye through the aperture of the light interceptor 106. In some embodiments, only light reflected from the fundus of the eye and passing through the pupil of the eye is received at the imaging sensor 104.

In some embodiments, the light interceptor 106 may be removable from the system 100. Alternatively or in addition, the light interceptor 106 can be shaped according to a shape of the pupil of the eye 102 of the subject. Alternatively or in addition to the light interceptor 106 being shaped according to a shape of the pupil of the eye 102 of the subject, in some embodiments, the aperture of the light interceptor 106 may be shaped according to the shape of the pupil of the eye 102 of the subject. In some embodiments where the aperture of the light interceptor 106 is shaped according to a shape of the pupil, the light interceptor 106 may advantageously comprise an optical device, such as a liquid crystal device LCD or a digital mirror device DMD. For example, the light interceptor 106 may comprise a matrix of individually controllable LCD or DMD pixel elements, which can function as an active spatial light modulator that is arranged to function as the aperture. The use of such an active spatial light modulator can allow the shape of the aperture to follow the shape of the image of the pupil in the image plane of the pupil. This allows the aperture, for example, to adopt an elliptical shape to follow the elliptical appearance of the pupil when observed from a non-perpendicular angle. The also allows the aperture, for example, to adopt a more complex shape to match the complex shape of the visible pupil due to partial occlusion. A partial occlusion may, for example, be caused by an eye lid or by eye lashes.

Alternatively or in addition to the aperture of the light interceptor 106 being shaped according to a shape of the pupil, the aperture of the light interceptor 106 may be sized according to a size of the pupil of the eye 102 of the subject. For example, the aperture of the light interceptor 106 may be adjustable to match the size of the pupil of the eye 102 of the subject.

Returning back to Figure 1, the system 100 further comprises one or more processors (or one or more control units) 108 operably coupled with the imaging sensor 104 and the light interceptor 106. The one or more processors 108 are configured to detect movement of the pupil of the eye 102 in the image acquired by the imaging sensor 104. For example, the movement of the pupil of the eye 102 can be detected with respect to the optical axis. The one or more processors 108 are also configured to control the light interceptor 106 to move (or actuate) relative to the optical axis in response to the detected movement of the pupil of the eye 102 to maintain the alignment (or correspondence, or coincidence) of the image of the pupil of the eye 102 with the aperture of the light interceptor 106. The light interceptor 106 can be moved dynamically. In addition to moving the light interceptor 106 relative to the optical axis, in some embodiments, the diameter of the aperture of the light interceptor 106 may also be dynamically adapted (for example, in the manner described earlier) for the imaging sensor 104 to acquire an optimal image.

In some embodiments, the one or more processors 108 may be configured to control the light interceptor 106 to maintain a lateral position of the image of the pupil of the eye 102 in alignment (or correspondence) with a lateral position of the aperture of the light interceptor 106. Alternatively or in addition, the one or more processors 108 may be configured to control the light interceptor 106 to maintain a longitudinal position of the image of the pupil of the eye 102 in alignment with a longitudinal position of the aperture of the light interceptor 106 (for example, in an intermediate image plane). In this way, the image of the pupil can be maintained in focus where the image of the pupil meets the aperture of the light interceptor 106.

Thus, in some embodiments, the one or more processors 108 may be configured to control the light interceptor 106 to move laterally (for example, perpendicularly) to the optical axis in response to the detected movement of the pupil of the eye 102 to maintain the alignment of the image of the pupil of the eye 102 with the aperture of the light interceptor 106. A larger translation of the face and/or the eye of the subject laterally to the optical axis will result in the misalignment (or decentering) of the image of the pupil of the eye 102 with the aperture of the light interceptor 106. This misalignment can thus be prevented by dynamically translating the position of the light interceptor 106 with respect to the optical axis. Alternatively or in addition to the lateral movement, in some embodiments, the one or more processors 108 may be configured to control the light interceptor 106 to move longitudinally along the optical axis in response to the detected movement of the pupil of the eye 102 to maintain the alignment of the image of the pupil of the eye 102 with the aperture of the light interceptor 106.

In some embodiments where the light interceptor 106 is controlled to move laterally to the optical axis, the one or more processors 108 may be configured to fix the light interceptor 106 in the longitudinal direction along the optical axis. In these embodiments, in addition to the lateral movement of the light interceptor 106, the one or more processors 108 may be configured to control movement of one or more lenses in the system 100 (for example, a longitudinal movement of the one or more lenses along the optical axis of the system 100) to maintain the focus of the image of the pupil where the image of the pupil meets the aperture of the light interceptor 106. In other embodiments where the light interceptor 106 moves laterally to the optical axis, the light interceptor 106 may also move longitudinally along the optical axis.

In some embodiments, in addition to the imaging sensor 104, light interceptor 106 and one or more processors 108 mentioned earlier, the system 100 may also comprise other components. For example, the system 100 may comprise one or more lenses, one or more beam splitters, one or more light polarizers, one or more light filters, one or more mirrors, one or more other optical components, one or more memories, one or more audio components (such as one or more speakers), one or more visual components (such as one or more display screens), or any other component, or any combination of components. Any one or more of the components of the system 100 may be operably coupled together via one or more wired or wireless pathways (which can, for example, be data/electrical/communication pathways, such as one or more buses).

As illustrated in Figure 1, for example, the system 100 may also comprise one or more imaging lenses 110 through which light reflected from the eye 102 of the subject passes, one or more beam splitters 112 for splitting light and/or one or more relay lenses 114 through which light reflected from the eye 102 of the subject passes according to some embodiments. The system 100 can, for example, comprise one or more imaging lenses 110 positioned along the optical axis, which is between the imaging sensor 104 and the eye 102. The one or more imaging lenses 110 can be positioned between the light interceptor 106 and the imaging sensor 104. After passing through the aperture of the light interceptor 106, the light reflected from the eye 102 of the subject passes through the one or more imaging lenses 110 to the imaging sensor 104. The one or more imaging lenses 110 focus the light reflected from the eye 102 of the subject that passes through the aperture of the light interceptor 106 on the imaging sensor 104. The one or more imaging lenses 110 may, for example, comprise one or more achromatic lenses, one or more achromatic lens combinations, or any other type of lenses, or any combination of lenses, suitable to focus light on the imaging sensor 104.

Alternatively or in addition, in some embodiments, the system 100 can comprise one or more beam splitters 112 angled to reflect light emitted by a light source (not illustrated). The one or more beam splitters 112 can, for example, reflect light emitted by a light source toward the eye 102 of the subject. The light that is then reflected from the eye 102 of the subject, including from the fundus of the eye, may pass through the one or more beam splitter 112 toward the light interceptor 106. The one or more beam splitters 112 may separate the imaging part of the system 100 and the illumination part of the system 100. The one or more beam splitters 112 may comprise, for example, one or more dichroic semi-transparent mirrors, or any other type of beam splitters, or any combination of beam splitters, suitable to operate in the manner described herein.

Alternatively or in addition, in some embodiments, the system 100 can comprise one or more relay lenses 114. The one or more relay lenses 114 can be positioned along the optical axis, which is between the imaging sensor 104 and the eye 102. The light reflected from the eye 102 passes through the one or more relay lenses 114 before reaching the light interceptor 106. The one or more relay lenses 114 can, for example, focus light reflected from the eye 102 on the light interceptor 106. The one or more relay lenses 114 may, for example, comprise one or more achromatic lenses, one or more achromatic lens combinations, or any other type of lens, or any combination of lenses, suitable to focus light reflected from the eye 102 on the light interceptor 106. In embodiments comprising one or more relay lenses 114 and one or more imaging lenses 110, as illustrated in Figure 1, the light interceptor 106 may be positioned between at least one of the relay lenses 114 and at least one of the imaging lenses 110.

Although not illustrated in Figure 1, in some embodiments, the system 100 can comprise a light source for illuminating the eye 102 of the subject with light. For example, the light source can be configured to provide an illumination beam (or emit light) towards the eye 102 of the subject or, more specifically, towards the fundus of the eye 102 of the subject. In some embodiments, a diameter of the illumination beam may be greater than a diameter of the pupil of the eye 102 of the subject.

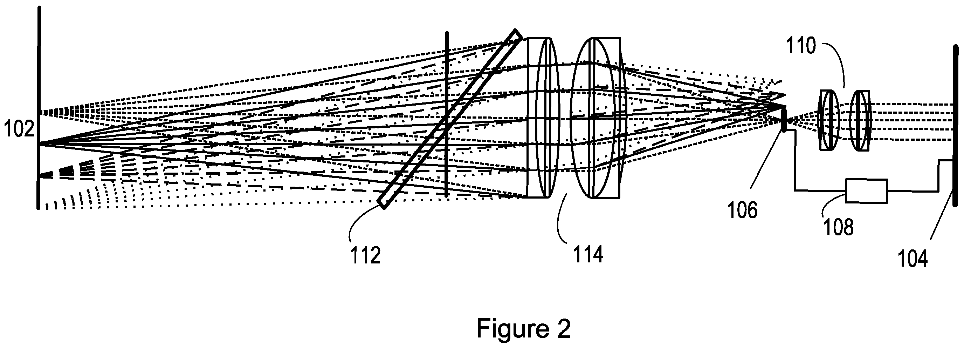

Figure 2 illustrates another example of the system 100 for imaging a fundus of an eye 102 of a subject (not depicted). While Figure 1 illustrates the path of light reflected from the posterior segment (for example, the fundus) of the eye 102 to the imaging sensor 104, Figure 2 illustrates the path of light reflected from the (potentially scattering) anterior segment (for example, the iris) of the eye 102 to the imaging sensor 104. As illustrated in Figure 2, the light reflected from the iris of the eye 102 is focused on the plane of the light interceptor 106. In some embodiments, the shape of the aperture of the light interceptor 106 may match the outline of the visible pupil.

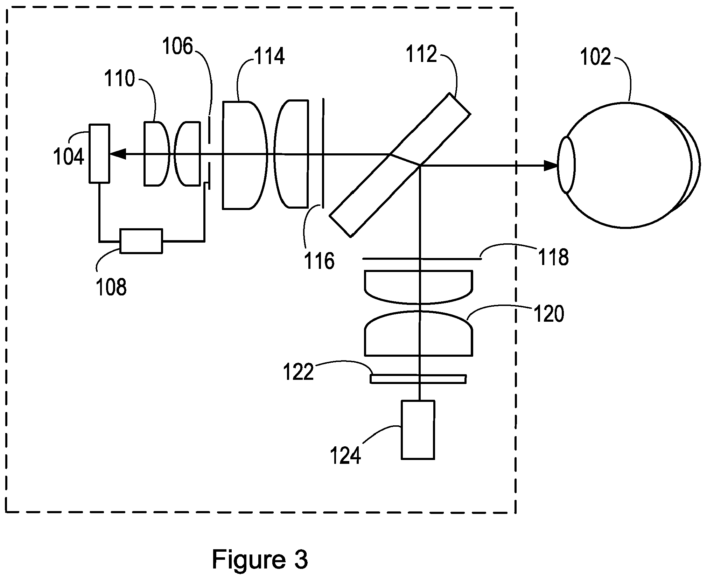

Figure 3 illustrates an example of the system 100 for imaging a fundus of an eye 102 of a subject (not depicted) according to another embodiment. The system 100 of Figure 3 illustrates an alternative arrangement of components to that illustrated in Figure 1. However, it will be understood that the components are operated in the same manner, as described earlier with reference to Figure 1. Although the system 100 will now be described with reference to the arrangement of components shown in Figure 3, it will be understood that other components and arrangements of components are possible.

With reference to Figure 3, the system 100 comprises an imaging sensor 104. The imaging sensor 104 is configured to be aimed towards the subject to acquire an image of the fundus of the eye 102 of the subject. As mentioned earlier, in some embodiments, the imaging sensor 104 may be configured to acquire the image of the fundus of the eye 102 at a remote distance from the eye 102. The system 100 also comprises a light interceptor 106, which is as described earlier with reference to Figure 1.

As illustrated in Figure 3, the light interceptor 106 is positioned on an optical axis between the imaging sensor 104 and the eye 102. The light interceptor 106 is positioned on the optical axis at a point at which an image of the pupil of the eye 102 aligns (or corresponds, or coincides) with the aperture of the light interceptor 106. In some embodiments, the light interceptor 106 may be positioned on the optical axis between the imaging sensor 104 and the eye 102 at the point at which the pupil of the eye is in focus in the system 100. The point at which the pupil is in focus in the system 100 is, for example, where a light beam reflected from the eye has the smallest diameter. In some embodiments, the point at which the pupil is in focus in the system 100 is at an image of the rim of the eye 102 (or at the image plane of the rim of the eye 102).

The light interceptor 106 is movable relative to the optical axis to selectively block light passing through the system 100 from the eye 102 to the imaging sensor 104. More specifically, the light interceptor 106 prevents the passage of light, except for the light passing through its aperture. In some embodiments, light passing through the system 100 from the outside of the pupil of the eye 102 is blocked by the light interceptor 106. The imaging sensor 104 of the system 100 can be configured to acquire the image of the fundus of the eye 102 of the subject by imaging the pupil of the eye through the aperture of the light interceptor 106. In some embodiments, only light reflected from the fundus of the eye and passing through the pupil of the eye is received at the imaging sensor 104. In some embodiments, the light interceptor 106 may be removable from the system 100. The light interceptor 106 may be shaped in the manner described earlier with reference to Figure 1.

Returning back to Figure 3, the system 100 further comprises one or more processors (or one or more control units) 108 operably coupled with the imaging sensor 104 and the light interceptor 106. The one or more processors 108 are configured to detect movement of the pupil of the eye 102 in the image acquired by the imaging sensor 104. The one or more processors 108 are also configured to control the light interceptor 106 to move relative to the optical axis in response to the detected movement of the pupil of the eye 102 to maintain the alignment (or correspondence, or coincidence) of the image of the pupil of the eye 102 with the aperture of the light interceptor 106. The one or more processors 108 may be configured to control the light interceptor 106 to move in any of the manners described earlier with reference to Figure 1. In addition to moving the light interceptor 106 relative to the optical axis, in some embodiments, the diameter of the aperture of the light interceptor 106 may also be dynamically adapted, as described earlier, for the imaging sensor 104 to acquire an optimal image.

In some embodiments, in addition to the imaging sensor 104, light interceptor 106 and one or more processors 108 mentioned earlier, the system 100 may also comprise other components, such as any of those described earlier with reference to Figure 1. As illustrated in Figure 3, for example, the system 100 may also comprise one or more imaging lenses 110 through which light reflected from the eye 102 of the subject passes, one or more beam splitters 112 for splitting light and/or one or more relay lenses 114 through which light reflected from the eye 102 of the subject passes according to some embodiments. The one or more imaging lenses 110, one or more beam splitters 112 and/or one or more relay lenses 114 of Figure 3 are arranged and operate in the same manner as described earlier with reference to Figure 1.

The system 100 of Figure 3 also comprises one or more light polarizers 116. The one or more light polarizers 116 can suppress unwanted specular reflections. The most dominant specular reflections generally occur on the outer surface of the cornea and are generally caused by the coaxial illumination of the system 100 for imaging the fundus. In some embodiments, the use of a linear polarizer in the imaging path may be combined with a light source that emits light that is polarized in the perpendicular direction.

As illustrated in Figure 3, in some embodiments, the system 100 can comprise a light source 124 for illuminating the eye 102 of the subject with light. For example, the light source 124 can be configured to provide an illumination beam (or emit light) towards the eye 102 of the subject or, more specifically, towards the fundus of the eye 102 of the subject. In some embodiments, a diameter of the illumination beam may be greater than a diameter of the pupil of the eye 102 of the subject. The light from the light source 124 is reflected by one or more beam splitters 112 into the eye 102 of the subject and the light is then reflected from the eye 102, including from the fundus of the eye. The light reflected from the eye 102 of the subject passes through the one or more beam splitters 112 toward the light interceptor 106 via the one or more light polarizers 116 and the one or more relay lenses 114. The one or more beam splitters 112 separate the imaging part of the system 100 and the illumination part of the system 100. The one or more beam splitters 112 may comprise, for example, one or more dichroic semi-transparent mirrors, or any other type of beam splitters, or any combination of beam splitters, suitable to operate in the manner described herein.

In some embodiments, the system 100 can comprise one or more further light polarizers 118, one or more further lenses 120 and/or one or more light filters 122. The one or more further light polarizers 118 can suppress unwanted specular reflections, for example, in the manner described earlier with respect to the one or more light polarizers 116. The one or more further light polarizers 118 can, for example, comprise one or more horizontal light polarizers. The one or more light polarizers 116 can be positioned orthogonal to the one or more further light polarizers 118.

The one or more further lenses 120 refract the light emitted from the light source 124 such that the imaged fundus area is illuminated. The one or more further lenses 120 can, for example, comprise a combination of positive and negative refracting or reflecting elements to efficiently transfer the light emitted from the light source to the imaged fundus area. The one or more light filters 122 can, for example, suppress an undesired part of the light that is emitted by the light source 124. The one or more light filters 122 can, for example, comprise one or more long pass filters in order to suppress a range of shorter wavelengths, which may not contribute diagnostically relevant information to the images acquired of the fundus by the imaging sensor 104. The one or more light filters 122 can, for example, comprise one or more high pass filters, such as to suppress harmful far-infrared radiation from the light source 124 according to some embodiments.

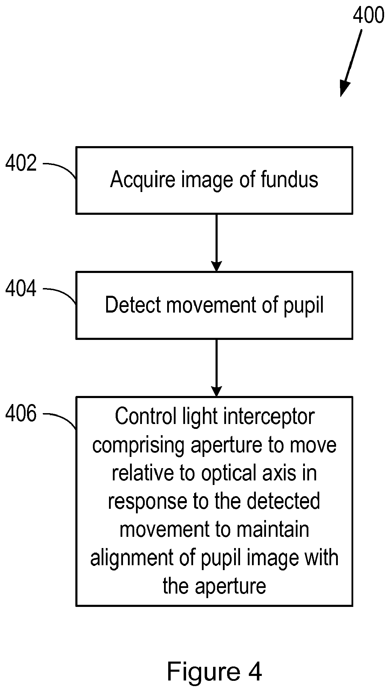

Figure 4 illustrates a method 400 of operating a system 100 to image a fundus of an eye 102 of a subject according to an embodiment. The method 100 is for use in operating a system 100 according to any of the embodiments described herein.

As illustrated in Figure 4, at block 402, an image of the fundus of the eye of the subject is acquired by the imaging sensor 104 that is aimed towards the subject. At block 404 of Figure 4, movement of the pupil of the eye is detected in the image acquired by the imaging sensor 104. The movement is detected by the one or more processors 108. At block 406 of Figure 4, the light interceptor 106 comprising the aperture is controlled by the one or more processors 108. As described earlier, the light interceptor 106 is positioned on an optical axis between the imaging sensor 104 and the eye 102 at a point at which an image of the pupil of the eye aligns (or corresponds, or coincides) with the aperture of the light interceptor 106 and the light interceptor 106 is movable relative to the optical axis to selectively block light passing through the system 100 from the eye 102 to the imaging sensor 104. The light interceptor 106 is controlled by the one or more processors 108 to move relative to the optical axis in response to the detected movement of the pupil of the eye 102 to maintain the alignment (or correspondence, or coincidence) of the image of the pupil of the eye 102 with the aperture of the light interceptor 106.

As mentioned earlier, the imaging sensor 104 of the system 100 described herein is configured to acquire an image of the fundus of the eye 102 of the subject. In some embodiments, the imaging sensor 104 of the system 100 may be configured to acquire a sequence of images of the fundus of the eye 102. In these embodiments, the sequence of images of the fundus of the eye 102 can be combined to form a single image of the fundus of the eye 102 with a larger field of view than the individual images in the sequence. For example, the field of view can be increased by stitching of the multiple images of the fundus of the eye 102 in the sequence in order to compose an image covering a wider fundus area. The one or more processors 108 of the system 100 may be configured to combine the sequence of images of the fundus in this way. It is possible to compose an image covering a wider fundus area by combining multiple images of the fundus since the subject is free to move their eye using the system described herein.

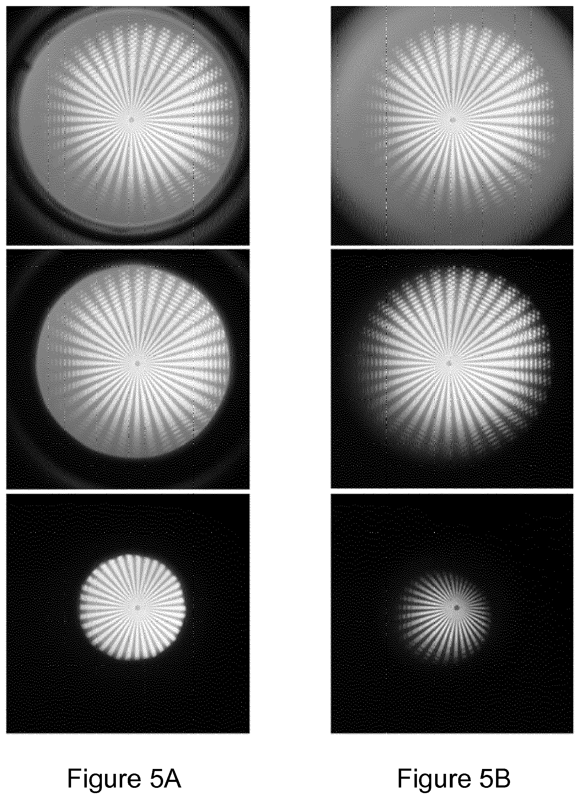

Figures 5A and 5B are images illustrating the contrast improvements achieved by a system 100 according to an embodiment. The images illustrated in Figures 5A and 5B are images acquired at the same exposure setting. Figure 5A illustrates a sequence of images acquired by the imaging sensor 104 while positioning the light interceptor 106 on the optical axis between the imaging sensor 104 and the eye 102 at the point at which the fundus of the eye 102 is in focus in the system 100, i.e. at the image plane of the fundus of the eye 102. As shown in Figure 5A, no contrast improvements in the images acquired of the fundus of the eye 102 are seen. Figure 5B illustrates a sequence of images acquired by the imaging sensor 104 while positioning the light interceptor 106 on the optical axis between the imaging sensor 104 and the eye 102 at the point at which the pupil of the eye is in focus in the system 100, such as at the image plane of the rim of the eye 102. As shown in Figure 5B, significant contrast improvements in the images acquired of the fundus of the eye 102 are seen.

There is therefore provided herein an improved system 100 for imaging a fundus of an eye 102 of a subject and also a method 400 of operating the system 100 to image a fundus of an eye 102 of a subject. As mentioned earlier, by positioning the light interceptor 106 on the optical axis between the imaging sensor 104 and the eye 102 at a point at which an image of the pupil of the eye 102 aligns with the aperture of the light interceptor 106 and controlling the light interceptor 106 to move relative to the optical axis in response to a detected movement of the pupil of the eye 102 to maintain the alignment of the image of the pupil of the eye 102 with the aperture of the light interceptor 106, only the desired light contribution from the fundus is allowed to be preserved and the stray light (for example, from rim of the eye 102) is blocked. In this way, the image contrast that is possible at a remote distance from the eye 102 is improved such that sharper (and thus higher quality) images can be acquired at the remote distance.

The system described herein can be used for unobtrusive (and, for example, automated) health monitoring on the basis of images captured of the fundus of the eye. As such, the system can be used by untrained users, such as general practitioners, nurse practitioners, and even the subject themselves, thereby increasing the availability of fundus imaging. The system can be used in the home (for example, in the bathroom) as part of a home-health monitoring system. The system may be used for early detection or monitoring of a physical or physiological condition or to follow deterioration. This can, for example be useful in respect of subjects suffering from retinopathy, such as that caused by diabetes or hypertension. In such applications, the acquired images can be made available to a medical professional to enable the early detection or monitoring in a remote manner, such as via the internet. In another application, the system may be used for remote spectral monitoring of metabolite concentrations dissolved in the blood, in order to report the concentrations to a user (for example, the subject themselves, an ophthalmologist, a medical professional, or any other user) or to store the concentrations as measurements as part of a health monitoring system.

There is further provided a computer program product comprising a computer readable medium, the computer readable medium having computer readable code embodied therein, the computer readable code being configured such that, on execution by a suitable computer or processor, the computer or processor is caused to perform the methods described herein.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A system (100) for imaging a fundus of an eye (102) of a subject, the system (100) comprising:
an imaging sensor (104) configured to be aimed towards the subject to acquire an image of the fundus of the eye (102) of the subject;
a light interceptor (106) comprising an aperture, wherein the light interceptor (106) is positioned on an optical axis between the imaging sensor (104) and the eye (102) at a point at which an image of the pupil of the eye (102) aligns with the aperture of the light interceptor (106) and wherein the light interceptor (106) is movable relative to the optical axis to selectively block light passing through the system from the eye (102) to the imaging sensor (106);
one or more processors (108) operably coupled with the imaging sensor (104) and the light interceptor (106), the one or more processors (108) configured to:
detect movement of the pupil of the eye (102) in the image acquired by the imaging sensor (104); and
control the light interceptor (106) to move relative to the optical axis in response to the detected movement of the pupil of the eye (102) to maintain the alignment of the image of the pupil of the eye (102) with the aperture of the light interceptor (106).

2. A system (100) as claimed in claim 1, wherein the one or more processors (108) are configured to control the light interceptor (106) to maintain one or more of:
a lateral position of the image of the pupil of the eye (102) in alignment with a lateral position of the aperture of the light interceptor (106); and
a longitudinal position of the image of the pupil of the eye (102) in alignment with a longitudinal position of the aperture of the light interceptor (106).

3. A system (100) as claimed in claim 1 or 2, wherein the one or more processors (108) are configured to control the light interceptor (106) to move laterally to the optical axis and/or longitudinally along the optical axis in response to the detected movement of the pupil of the eye (102) to maintain the alignment of the image of the pupil of the eye (102) with the aperture of the light interceptor (106).

4. A system (100) as claimed in any one of the preceding claims, wherein light passing through the system (100) from the outside of the pupil of the eye (102) is blocked by the light interceptor (106).

5. A system (100) as claimed in any one of the preceding claims, wherein the imaging sensor (104) is configured to acquire the image of the fundus of the eye (102) of the subject by imaging the pupil of the eye (102) through the aperture of the light interceptor (106).

6. A system (100) as claimed in any one of the preceding claims, the system (100) further comprising:
a light source (124) configured to provide an illumination beam towards the fundus of the eye of (102) the subject, wherein a diameter of the illumination beam is greater than a diameter of the pupil of the eye (102) of the subject.

7. A system (100) as claimed in any one of the preceding claims, wherein the light interceptor (106) is positioned on the optical axis between the imaging sensor (104) and the eye (102) at the point at which the pupil of the eye (102) is in focus in the system (100).

8. A system (100) as claimed in any one of the preceding claims, wherein only light reflected from the fundus of the eye (102) and passing through the pupil of the eye (102) is received at the imaging sensor (104).

9. A system (100) as claimed in any one of the preceding claims, wherein the imaging sensor (104) is configured to acquire the image of the fundus of the eye (102) at a remote distance from the eye (102).

10. A system (100) as claimed in any one of the preceding claims, the system (100) further comprising:
one or more relay lenses (114) positioned along the optical axis between the imaging sensor (104) and the eye (102); and/or
one or more imaging lenses (110) positioned along the optical axis between the imaging sensor (104) and the eye (102).

11. A system (100) as claimed in claim 10, wherein the light interceptor (106) is positioned between at least one of the relay lenses (114) and at least one of the imaging lenses (110).

12. A system (100) as claimed in any one of the preceding claims, wherein the imaging sensor (104) is configured to:
acquire a sequence of images of the fundus of the eye (102); and
combine the sequence of images of the fundus of the eye (102) to form a single image of the fundus of the eye (102) with a larger field of view than the individual images in the sequence.

13. A system (100) as claimed in any one of the preceding claims, wherein the light interceptor (106) is removable from the system (100) and shaped according to a shape of the pupil of the eye (102) of the user.

14. A method (400) for operating a system (100) to image a fundus of an eye (102) of a subject, the method (400) comprising:
acquiring (402), by an imaging sensor (104) aimed towards the subject, an image of the fundus of the eye (102) of the subject;
detecting (404), by one or more processors (108), movement of the pupil of the eye (102) in the image acquired by the imaging sensor (104); and
controlling (406), by one or more processors (108), a light interceptor (106) comprising an aperture, wherein the light interceptor (106) is positioned on an optical axis between the imaging sensor (104) and the eye (102) at a point at which an image of the pupil of the eye (102) aligns with the aperture of the light interceptor (106) and the light interceptor (106) is movable relative to the optical axis to selectively block light passing through the system (100) from the eye (102) to the imaging sensor (104), and
wherein the light interceptor (106) is controlled by the one or more processors (108) to move relative to the optical axis in response to the detected movement of the pupil of the eye (102) to maintain the alignment of the image of the pupil of the eye (102) with the aperture of the light interceptor (106).

15. A computer program product comprising a computer readable medium, the computer readable medium having computer readable code embodied therein, the computer readable code being configured such that, on execution by a suitable computer or processor, the computer or processor is caused to perform the method as claimed in claim 14.
